# EUROPÄISCHE PATENTSCHRIFT

(11) **EP 2 583 700 B1**
(45) Veröffentlichungstag und Bekanntmachung des Hinweises auf die Patenterteilung: **02.12.2020**
(21) Anmeldenummer: 11185961.7
(22) Anmeldetag: 20.10.2011
(51) Int. Cl.: A61M 1/36

(54) **Starten einer Hämodialyse**
starting haemodialysis
démarrage d'une hémodialyse

(43) Veröffentlichungstag der Anmeldung: 24.04.2013
(60) Teilanmeldung: 19183281.5
(73) Patentinhaber: D.Med Consulting GmbH, 20359 Hamburg (DE)
(72) Erfinder: Breuch, Gerd, 53844 Troisdorf (DE); Biermann, Frank, 22455 Hamburg (DE); Yanagimoto, Yoji, 1030 Schaerbeek (BE)
(74) Vertreter: terpatent Patentanwälte ter Smitten Eberlein-Van Hoof Rütten Daubert

(56) Entgegenhaltungen:
- EP-A1- 1 457 218
- DE-B4- 19 655 226
- US-A- 5 932 103
- US-A1- 2007 118 064
- US-A1- 2009 101 576

## Beschreibung

Die Erfindung bezieht sich auf ein Hämodialysegerät zum Starten einer Hämodialyse.

Ein typisches Hämodialysegerät weist eine Dialysatseite und eine Blutseite auf. Zu der Dialysatseite zählt eine Dialysatquelle, die das Dialysat zur Verfügung stellt, und zwar entweder in Form von durch das Hämodialysegerät mit Additiven versetzten Dialysewasser oder in Form eines Dialysat-Vorrats. Ferner zählt die Dialysatkammer eines eine Membran aufweisenden Dialysators und eine Dialysatpumpe, die das Dialysat von der Dialysatquelle zu der Dialysatkammer pumpt, zu der Dialysatseite. Schließlich sind auf der Dialysatseite zwischen der Dialysatkammer und einem Abfalltank bzw. einem Abfallablauf eine Saugpumpe und ein Dialysatdruck-Sensor zwischen der Saupumpe und der Blutkammer vorgesehen. Zu der Blutseite zählen die Blutkammer des Dialysators, sowie eine venöse Leitung und eine arterielle Leitung, die beide an die Blutkammer angeschlossen sind.

Ferner ist eine Vorfüllflüssigkeits-Quelle vorgesehen, die Vorfüllflüssigkeit für das Vorfüllen der venösen und der arteriellen Leitung zur Verfügung stellt. Die Vorflüssigkeits- Quelle kann von der Dialysatquelle gebildet werden, kann jedoch auch eine separate Vorflüssigkeits- Quelle sein, beispielsweise ein Beutel mit Kochsalzlösung,

Vor dem Anlegen der venösen Leitung und der arteriellen Leitung an den Patienten werden die beiden blutseitigen Leitungen mit Vorfüllflüssigkeit vorgefüllt, wie dies aus DE 196 55 226 B4 bekannt ist, in der als Vorfüllflüssigkeit Dialysat verwendet wird. Nach dem Vorfüllen der blutseitigen Leitungen mit der Vorfüllflüssigkeit werden die Leitungen an den Patienten angelegt, das heißt, es wird die arterielle Leitung mit ihrer Kanüle an eine Patienten-Arterie und die venöse Leitung mit ihrer Kanüle an eine Patienten-Vene angeschlossen. Bevor die eigentliche Blutreinigung beginnt, wird die Vorfüllflüssigkeit aus den Leitungen wieder entfernt, um die Dialyse mit einem unverdünnten Blutvolumen zu beginnen, um also einen sogenannten "clean start" zu ermöglichen. Die Vorfüllflüssigkeits-Entfernung erfolgt beispielsweise durch Ultrafiltration, bei der die Vorfüllflüssigkeit aus der arteriellen und der venösen Leitung über den Dialysator entfernt wird, indem die Vorfüllflüssigkeit durch die Dialysator-Membran hindurch durch die Saugpumpe abgepumpt, also ultrafiltriert wird. Das Abpumpen kann beispielsweise volumengesteuert erfolgen. Hierbei kann jedoch nicht ausgeschlossen werden, dass ein zu großes Flüssigkeitsvolumen aus den vorgefüllten Leitungen abgepumpt wird, d.h., dass nach dem Abpumpen der Vorfüllflüssigkeit auch noch das nachfließende Patientenblut unerwünschter Weise ultrafiltriert wird. Aufgabe der Erfindung ist es demgegenüber, ein Hämodialysegerät zum Starten einer Hämodialyse zu schaffen, bei dem bei der Ultrafiltration der Vorfüllflüssigkeit aus den blutseitigen Leitungen eine Ultrafiltration des Patientenblutes zuverlässig verhindert wird.

Ein Hämodialysegerät ist auch aus EP 1 457 218 A1 bekannt.

Dieses Hämodialysegerät entfernt die Vorfüllflüssigkeit durch Ultrafiltration aus der arteriellen und venösen Leitung durch die Dialysator-Membran in die Dialysatkammer des Dialysators. Die Steuerung basiert auf einer Bestimmung des entfernten Flüssigkeitsvolumen oder der Zeitdauer des entsprechenden Prozesschrittes.

Diese Aufgabe wird erfindungsgemäß gelöst mit einem Hämodialysegerät den Merkmalen des Patentanspruches 1.

Bei dem erfindungsgemäßen Hämodialysegerät zum Starten einer Hämodialyse wird nach dem Vorfüllen der venösen und der arteriellen Leitung mit der Vorfüllflüssigkeit die Vorfüllflüssigkeit durch die Dialysator-Membran hindurch in die Dialysatkammer des Dialysators mit einer beispielsweise konstanten Entfernungs-Durchtrittsrate durch die Saugpumpe ultrafiltriert, also zur Dialysatseite zurückgepumpt. Die Saugpumpe ist beispielsweise eine Abfallpumpe und/oder eine separate Ultrafiltrationspumpe, die parallel zu der Abfallpumpe einer Bilanzierpumpe angeordnet ist. Während der gesamten Dauer der Vorfüllflüssigkeits- Entfernung wird der Fluiddruck auf der Dialysatseite des Dialysators durch den Dialysatdruck-Sensor ermittelt und überwacht, der zwischen der Saugpumpe und der Dialysatkammer des Dialysators angeordnet ist. Der Dialysatdruck- Sensor ermittelt also den Saugdruck der Saugpumpe. Sobald der durch den Dialysatdruck-Sensor gemessene Fluid- bzw. Saugdruck einen festgelegten Blutankufts-Grenzdruck durch- bzw. unterschreitet, wird die Vorfüllflüssigkeits- Entfernung zwangsweise gestoppt.

Die Viskosität des menschlichen Blutes bzw. des Patientenblutes ist erheblich höher als die Viskosität der Vorfüllflüssigkeit, unabhängig davon, ob die Vorfüllflüssigkeit in Form einer Kochsalzlösung oder in Form von Dialysat vorliegt. In dem Moment, in dem das Patientenblut die Dialysator-Membran erreicht, erhöht sich der Flusswiderstand durch die Dialysator- Membran hindurch, so dass auf der Dialysatseite der Druck unter den festgelegten Blutankunfts-Grenzdruck abfällt, solange die für die Vorfüllflüssigkeits- Entfernung verantwortliche Saugpumpe weiter arbeitet. Aus dem Druckabfall kann also darauf geschlossen werden, dass das Patientenblut an der Dialysator-Membran angekommen ist. Der Blutankunfts-Grenzdruck liegt unter dem Druck, der auf der Dialysatseite während des Abpumpens der Vorfüllflüssigkeit anliegt, liegt jedoch über dem Druck, der bei Ankunft des Patientenblutes an der Dialysator-Membran anliegt. Das Unterschreiten des Blutankunfts-Grenzdrucks ist also ein sicheres Indiz dafür, dass die Vorfüllflüssigkeit aus der venösen und der arteriellen Leitung nahezu vollständig abgepumpt ist. Selbst wenn also eine Volumensteuerung bei der Vorfüllflüssigkeits- Entfernung ein weiteres Abpumpen von der Blutseite noch erlaubt hätte, wird bei Unterschreitung des festgelegten Blutankufts-Grenzdrucks die Vorfüllflüssigkeits- Entfernung vorzeitig beendet. Hierdurch wird zuverlässig verhindert, dass das Patientenblut in dieser Behandlungsphase unerwünschter Weise ultrafiltriert wird.

Unter einer Hämodialyse ist vorliegend jede Art der extrakorporalen Blutbehandlung zu verstehen.

Gemäß einer bevorzugten Ausgestaltung wird nach dem Stoppen der Vorfüllflüssigkeits- Entfernung der Dialysemodus mit einer anderen Durchtrittsrate gestartet, wobei die Dialysemodus-Durchtrittsrate niedriger, gleich Null oder in entgegengesetzte Richtung gerichtet ist als die Dialysatentfernungs- Durchtrittsrate. Mit Durchtrittrate ist vorliegend stets die Flussrate gemeint, mit der Flüssigkeit durch die Dialysator-Membran hindurchtritt. Normalerweise beginnt der Dialysemodus, womit die tatsächliche Blutreinigung gemeint ist, mit einer Durchtrittsrate von Null, so dass zunächst weder eine Ultrafiltration des Patientenblutes noch eine Einleitung von Dialysat in den blutseitigen Kreislauf vollzogen wird.

Vorzugsweise wird die Vorfüllflüssigkeits- Entfernung zwangsweise beendet, wenn eine vorgegebene Grenzmenge an Flüssigkeit über den Dialysator ultrafiltriert wurde. Die Vorfüllflüssigkeits- Entfernung wird also standardmäßig über den Dialysatdruck gesteuert, also beendet, sobald der Dialysatdruck den festgelegten Blutankufts-Grenzdruck unterschreitet. Zur Sicherheit wird die Vorfüllflüssigkeits- Entfernung auch bei Nicht-Unterschreitung des Blutankunfts-Grenzdrucks beendet, sobald eine vorgegebene maximale Grenzmenge an Dialysat über den Dialysator bereits ultrafiltriert wurde. Bei Ausfall der Dialysatdruck- gesteuerten Vorfüllflüssigkeits- Entfernung ist auf diese Weise sichergestellt, dass höchstens eine sehr geringe Menge an Patientenblut vor der Beendigung der Vorfüllflüssigkeits- Entfernung unerwünschter Weise ultrafiltriert wird.

Vorzugsweise wird während der Vorfüllflüssigkeits- Entfernung die Blutpumpe, die einer der beiden blutseitigen Leitungen, in der Regel der arteriellen Leitung, zugeordnet ist, mit einer Pumprate betrieben, die eine ungefähr gleichzeitige Blutankunft aus den beiden Leitungen an der Dialysator- Membran gewährleistet. Für den Fall, dass die arterielle Leitung einerseits und die venöse Leitung andererseits jeweils ungefähr das gleiche Gesamt- Innenvolumen aufweisen, würde die Pumprate der Blutpumpe ungefähr 50 % der Pumprate der Saugpumpe betragen, damit eine gleichzeitige Blutankunft des Patientenblutes aus beiden Leitungen in dem Dialysator realisiert wird.

Vorzugsweise ist die Dialysatpumpe zwangsweise mit einer Abfallpumpe zu einer Bilanzierpumpe gekoppelt, beispielsweise mechanisch gekoppelt. Hierdurch wird mechanisch sichergestellt, dass die Dialysatpumpe jeweils exakt dieselbe Flüssigkeitsmenge in Richtung Dialysator pumpt, wie die Abfallpumpe aus Richtung Dialysator abpumpt. Im Falle einer Bilanzierpumpe ist eine separate Ultrafiltrationspumpe vorgesehen, die fluidisch parallel zu der Abfallpumpe angeordnet ist. Die Ultrafiltrationspumpe wird beim Vorfüllen der Leitungen mit Vorfüllflüssigkeit bzw. mit Dialysat im Gegenstrom betrieben, d.h. sie pumpt einen Teil der von der Abfallpumpe stromabwärts gepumpten Flüssigkeit über einen Bypass zurück zur Einlassseite der Abfallpumpe. Hierbei liegt die Pumprate der Ultrafiltrationspumpe stets unter der Pumprate der Abfallpumpe, damit nicht bereits verbrauchtes Dialysat rückwärts in den Dialysator gedrückt werden kann. Bei der Vorfüllflüssigkeits- Entfernung wird die Ultrafiltrationspumpe als Saugpumpe im Parallelstrom betrieben, d.h. sie pumpt in die gleiche Richtung wie die Abfallpumpe.

Gemäß einer bevorzugten Ausgestaltung ist im Verlauf der venösen Leitung eine Luftfalle angeordnet, wobei während der Vorfüllflüssigkeits-Entfernung der Flüssigkeitspegel in der Luftfallenkammer oberhalb einer Einlassöffnung einer Einlaufleitung gehalten wird. Die Luftfalle weist eine oben angeordnete Einlaufleitung auf, durch die die Flüssigkeit, die in Luftfallen-Betriebsrichtung von dem. Dialysator aus in Richtung Patient fließt, in die Luftfallenkammer einläuft. Der Flüssigkeitspegel in der Luftfallenkammer kann bei Betrieb in Luftfallen-Betriebsrichtung unterhalb oder oberhalb der Einlaufleitung eingepegelt werden. Während der Vorfüllflüssigkeits- Entfernung strömt das Dialysat in der venösen Leitung jedoch entgegen der Betriebsrichtung der Luftfalle. Damit die Flüssigkeit während der Vorfüllflüssigkeits- Entfernung entgegen der Betriebsrichtung durch die Luftfalle gepumpt bzw. gesaugt werden kann, muss der Flüssigkeitspegel in der Luftfalle in jedem Fall oberhalb der Einlassöffnung der Einlaufleitung eingepegelt werden.

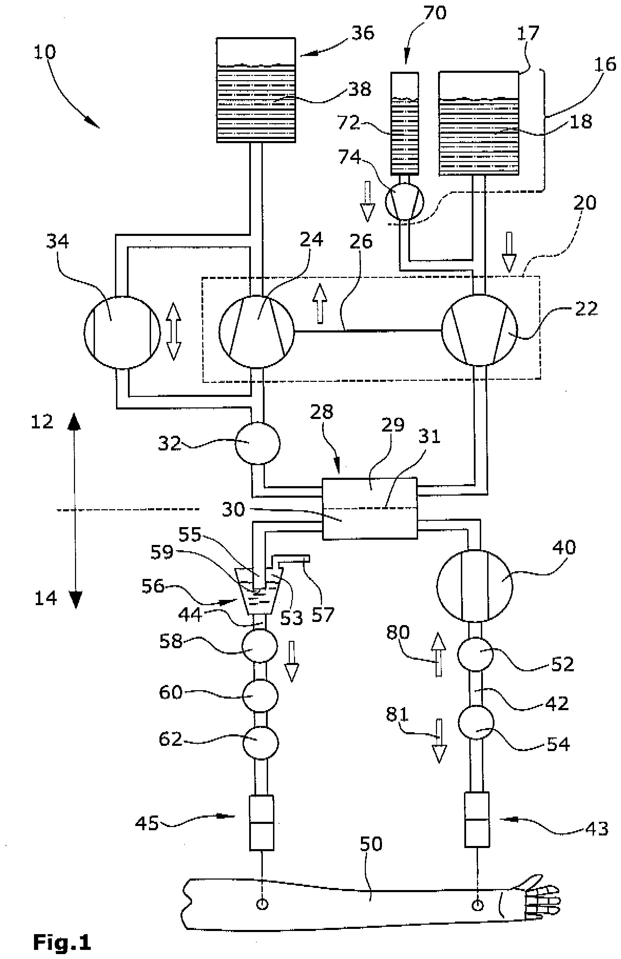

Im Folgenden wird ein Ausführungsbeispiel der Erfindung anhand der Zeichnung näher erläutert.

Die Figur zeigt schematisch ein Hämodialysegerät zur Durchführung einer Hämodialyse.

In der Figur ist schematisch ein Hämodialysegerät 10 dargestellt, das funktional in eine Dialysatseite 12 und eine Blutseite 14 unterteilt werden kann. Die Grenze zwischen der Dialysatseite 12 und der Blutseite 14 wird von einer Dialyse-Membran 31 in einem Dialysator 28 gebildet. Die Dialyse-Membran 31 trennt in dem Dialysator 28 eine Dialysatkammer 29 von einer Blutkammer 30.

Die Dialysatseite 12 weist eine Vorfüllflüssigkeits-Quelle 16 auf, die die Vorfüllflüssigkeit zur Verfügung stellt. Vorliegend ist die Vorflüssigkeits-Quelle 16 eine Dialysatquelle 16, die Dialysat für die Dialyse zur Verfügung stellt. Die Vorfüllflüssigkeits- und Dialysatquelle 16 wird von einem Dialysewasser- Tank 17 mit Dialysewasser 18 und einem Additiv-Tank 70 mit einem Dialysat- Additiv 72 gebildet. Dem Additiv-Tank 70 ist eine Additiv-Pumpe 74 nachgeordnet, die das Additiv 72 wohldosiert dem Dialysewasser-Strom zuführt. Es können auch mehrere Additiv-Tanks vorgesehen sein, wobei als Additiv insbesondere Elektrolyte und Puffersubstanzen vorgesehen sind. Das Dialysewasser kann, alternativ zu dem Dialysewasser-Tank 17, auch von einer (nicht dargestellten) Wasseraufbereitungs-Vorrichtung zur Verfügung gestellt werden, in der aus Trinkwasser aus dem öffentlichen Leitungsnetz das Dialysewasser hergestellt wird.

Das Dialysat aus der Vorfüllflüssigkeits-Quelle 16 wird durch eine Dialysatpumpe 22 in die Dialysatkammer 29 des Dialysators 28 gepumpt. Von der Dialysatkammer 29 aus verläuft eine Leitung zu der Abfallpumpe 24, die das Dialysat aus der Dialysatkammer 29 in einen Abfalltank 36 pumpt, in dem das verbrauchte Dialysat 38 gespeichert wird. Im Verlauf der Leitung zwischen der Dialysatkammer 29 und der Abfallpumpe 24 ist ein Dialysatdruck-Sensor 32 angeordnet, der den Druck der Dialysat-Flüssigkeit in diesen Leitungsabschnitt ermittelt. Fluidisch parallel zu der Abfallpumpe 24 ist eine Ultrafiltrationspumpe 34 angeordnet, die in beiden Richtungen betrieben werden kann, also im Gegenstrom und im Parallelstrom zu der Abfallpumpe 24 betrieben werden kann. Wenn die Ultrafiltrationspumpe 34 im Parallelstrom betrieben wird, bildet sie eine Saugpumpe 34, da sie dann Flüssigkeit von der Blutseite 14 durch die Membran 31 hindurch zur Dialysatseite 12 hinabsaugt.

Die Dialysatpumpe 22 und die Abfallpumpe 24 sind mechanisch durch eine mechanische Verbindung 26 miteinander verbunden, so dass die Pumpraten der Dialysatpumpe 22 und der Abfallpumpe 24 stets absolut identisch sind. Auf diese Weise bilden die Dialysatpumpe 23 und die Abfallpumpe 24 eine sogenannte Bilanzierpumpe 20. Die mechanische Verbindung 26 kann beispielsweise dadurch realisiert sein, dass die Bilanzierpumpe 20 als Membranpumpe ausgebildet ist, wobei die eine Seite der Pumpenmembran die Pumpkammer der Dialysatpumpe 22 und die andere Seite der Pumpenmembran die Pumpkammer der Abfallpumpe 24 bildet.

Auf der Blutseite der Dialysator-Membran 31 befindet sich die Blutkammer 30 des Dialysators 28, in die eine arterielle Leitung 42 und eine venöse Leitung 44 münden. Im Verlauf der arteriellen Leitung 42 ist eine Blutpumpe 40 angeordnet, deren Pumprichtung und Pumprate von einer Gerätesteuerung gesteuert werden. Die arterielle Leitung 42 weist an ihrem freien Ende eine arterielle Kanüle 43 und die venöse Leitung 44 an ihrem Ende eine venöse Kanüle 45 auf. Die Kanülen 43,45 dienen der Verbindung der beiden Leitungen 42,44 mit entsprechenden Blutgefäßen des Patienten 50. Zwischen der arteriellen Kanüle 43 und der Blutpumpe 40 sind ein arterieller Drucksensor 52 und eine arterielle elektrische Schlauchklemme 54 angeordnet.

Zwischen der Blutkammer 30 des Dialysators 28 und der venösen Kanüle 45 sind im Verlauf der venösen Leitung 44 eine Luftfalle 56, ein venöser Drucksensor 58, ein Luftsensor 60 mit integrierter Schlauchklemme und ein fotometrischer Farbsensor 62 angeordnet. Die Luftfalle 56 weist oben eine Flüssigkeits-Einlaufleitung 55 auf, die mit ihrer nach unten gerichteten Einlassöffnung 59 von oben in die Luftfallenkammer 53 hineinragt. Ferner weist die Luftfalle 56 einen Steuerluft-Anschluss 57 auf, über den durch das Hämodialysegerät die Höhe des Flüssigkeitspegels in der Luftfallenkammer 53 gesteuert bzw. geregelt wird. Der Drucksensor kann an eine Luftleitung angeschlossen sein, die in die Luftfalle mündet.

Bei Inbetriebnahme des Hämodialysegeräts 10 sind die Kanülen 43,45 zunächst noch nicht an den Patienten 50 angeschlossen, und sind auch nicht miteinander verbunden. Das Vorfüllen der arteriellen und venösen Leitung 42,44 mit Vorfüllflüssigkeit in Form von Dialysat erfolgt dadurch, dass die Bilanzierpumpe 20 in Betrieb genommen wird, wobei gleichzeitig die Ultrafiltrationspumpe 34 im Gegenstrom arbeitet. Es wird auf diese Weise in der Summe mehr Dialysat in die Dialysatkammer 29 gepumpt, als aus der Dialysatkammer 29 abgepumpt wird. Hierdurch wird Vorfüllflüssigkeit in Form von Dialysat in dem Dialysator 28 durch die Dialysator-Membran 31 hindurch in die beiden blutseitigen Leitungen 42,44 gepumpt, und zwar mit einer Durchtrittsrate, die exakt der Pumprate der Ultrafiltrationspumpe 34 entspricht. Die Blutpumpe 40 pumpt hierbei in Richtung der arteriellen Kanüle 43, also in Gegenrichtung 81, mit ungefähr der halben Pumprate der Ultrafiltrationspumpe 34, so dass die arterielle Leitung 42 und die venöse Leitung 44 jeweils mit derselben Flussrate gefüllt werden. Die Pumprate der Blutpumpe 40 ist so gewählt und gesteuert, dass beide Leitungen 42,44 ungefähr zum selben Zeitpunkt vollständig mit Vorfüllflüssigkeit in Form von Dialysat vorgefüllt sind. Sobald die beiden Leitungen 42,44 vollständig mit Vorfüllflüssigkeit bzw. Dialysat vorgefüllt sind, werden alle Pumpen 20,34,40 angehalten und werden die arterielle Leitung 42 mit ihrer Kanüle 43 einerseits und die venöse Leitung 44 mit ihrer Kanüle 45 andererseits an entsprechende Blutgefäße des Patienten 50 angeschlossen.

Anschließend wird die kontinuierliche Vorfüllflüssigkeits- Entfernung manuell durch eine Bedienperson ausgelöst. Bei der Vorfüllflüssigkeits-Entfernung wird die Ultrafiltrationspumpe 34 in Parallelstrom betrieben, das heißt, die Ultrafiltrationspumpe 34 pumpt mit einer Entfernungs-Durchtrittsrate in Richtung Abfalltank 36. Im Parallelstrom betrieben bildet die Ultrafiltrationspumpe nun eine Saugpumpe 34. Gleichzeitig pumpt die Blutpumpe 40 in Richtung Dialysator 28, also in ihrer Betriebsrichtung 80, und zwar mit einer Pumprate, die so gewählt ist, dass die Vorfüllflüssigkeit aus der arteriellen Leitung 42 und der venösen Leitung 44 symmetrisch abgepumpt wird. Auf diese Weise wird zum Einen die Vorfüllflüssigkeit aus den beiden Leitungen 42,44 entfernt, wobei zum Anderen die beiden Leitungen 42,44 gleichzeitig mit dem nachfolgenden Patientenblut aufgefüllt werden. Die Pumprate der Blutpumpe 40 ist so gewählt, dass die Blutankunft aus beiden Leitungen in der Blutkammer 30 annähernd gleichzeitig erfolgt. Das Verhältnis der Blutpumpen-Pumprate zur Saugpumpen-Pumprate entspricht daher ungefähr dem Verhältnis des Leitungsvolumens der arteriellen Leitung 42 zum Gesamtvolumen der beiden blutseitigen Leitungen 42,44 zusammen.

Während der Vorfüllflüssigkeits- Entfernung wird der Flüssigkeitspegel in der Luftfallenkammer 53 möglichst hoch eingestellt, mindestens jedoch so hoch eingestellt, dass der Flüssigkeitspegel stets oberhalb der Einlassöffnung 59 liegt. Die Steuerung der Höhe des Flüssigkeitspegels in der Luftfallenkammer 53 erfolgt durch das Hämodialysegerät 10, und konkret mit Hilfe von Steuerluft über den Steuerluft-Anschluss 57.

Während der Vorfüllflüssigkeits- Entfernung wird mit Hilfe des Dialysatdruck-Sensors 42 ständig der Fluiddruck auf der Dialysatseite ermittelt. Sobald der gemessene Fluiddruck einen festgelegten Blutankufts-Grenzdruck unterschreitet, wird die Vorfüllflüssigkeits-Entfernung gestoppt. Die Viskosität von Vorfüllflüssigkeit in Form von Dialysat oder einer Kochsalzlösung ist deutlich geringer als die von Patientenblut. Sobald Patientenblut die Dialysator-Membran 31 erreicht, steigt der Flusswiderstand und sinkt der von dem Dialysatdruck-Sensor 32 ermittelte Fluiddruck unter den Blutankunfts-Grenzdruck, so dass hierdurch ein sicheres Indiz für die Ankunft des Patientenblutes in dem Dialysator 28 vorliegt. In diesem Moment wird die Vorfüllflüssigkeits-Entfernung automatisch gestoppt.

Während der Vorfüllflüssigkeits- Entfernung kann, muss jedoch nicht, die Bilanzierpumpe 20 pumpen. Während der gesamten Vorfüllflüssigkeits-Entfernung wird aus der bekannten Pumprate der Saugpumpe 34 die ultrafiltrierte Flüssigkeitsmenge aufsummiert. Sobald die aufsummierte Flüssigkeitsmenge, die durch die Saugpumpe 34 abgepumpt wurde, eine vorgegebene Grenzmenge überschreitet, wird die Vorfüllflüssigkeits-Entfernung zwangsweise beendet. Hierdurch wird sichergestellt, dass bei einem defekten Dialysatdruck-Sensor 32 die Vorfüllflüssigkeits- Entfernung gestoppt wird, bevor nennenswerte Mengen an Patientenblut in dem Dialysator ultrafiltriert werden. Selbstverständlich kann als Standardverfahren auch umgekehrt eine Mengensteuerung gewählt werden, wobei als Ausfallsicherung dann die druckkontrollierte Beendigung der Vorfüllflüssigkeits- Entfernung dient.

Nach dem Stoppen der Vorfüllflüssigkeits- Entfernung wird die eigentliche Blutreinigung gestartet, wobei die Dialysat-Durchtrittsrate durch die Dialysator-Membran 31 zunächst gleich Null ist.

## Patentansprüche

1. Hämodialysegerät (10), das aufweist:
eine Vorfüllflüssigkeits- Quelle (16), die Vorfüllflüssigkeit zur Verfügung stellt,
eine Dialysatseite (12) mit einer Dialysatkammer (29) eines eine Membran (31) aufweisenden Dialysators (28), einer Dialysatpumpe (22), die Dialysat zu der Dialysatkammer (29) pumpt, einer Saugpumpe (34), die das Dialysat oder die Vorfüllflüssigkeit von der Dialysatkammer (29) weg pumpt, und einem Dialysatdruck-Sensor (32) zwischen der Dialysatkammer (29) und der Abfallpumpe (24), und
eine Blutseite (14) mit einer arteriellen Leitung (42), einer Blutpumpe (40), einer Blutkammer (30) des Dialysators (28) und einer venösen Leitung (44),
wobei das Hämodialysegerät (10)
bei mit Vorfüllflüssigkeit aus der Vorfüllflüssigkeits-Quelle (16) vorgefüllter arterieller und venöser Leitung (42,44),
bei jeweils an einen Patienten (50) angelegter arterieller und venöser Leitung (42,44),
und nach dem manuellen Auslösen der Vorfüllflüssigkeits-Entfernung
die folgenden Verfahrensschritte zum Starten einer Hämodialyse ausführt:
Vorfüllflüssigkeits-Entfernung durch Ultrafiltration der Vorfüllflüssigkeit aus der arteriellen und venösen Leitung (42,44) durch die Dialysator- Membran (31) in die Dialysatkammer (29) des Dialysators (28) durch die Saugpumpe (34), und
während der Vorfüllflüssigkeits- Entfernung: ständige Bestimmung des Fluiddrucks durch den Dialysatdruck- Sensor (32), wobei die Vorfüllflüssigkeits- Entfernung automatisch gestoppt wird, wenn der von dem Dialysatdruck- Sensor (32) gemessene Fluiddruck einen festgelegten Blutankunfts- Grenzdruck unterschreitet.

2. Hämodialysegerät (10) nach Anspruch 1, mit dem Verfahrensschritt, nach dem Stoppen der Vorfüllflüssigkeits-Entfernung: Start des Dialysemodus durch das Hämodialysegerät (10) mit einer Durchtrittsrate, die anders ist als die Vorfüllflüssigkeits- Entfernungs-Durchtrittsrate.

3. Hämodialysegerät (10) nach einem der vorangegangenen Ansprüche, wobei die Vorfüllflüssigkeits-Entfernung durch das Hämodialysegerät (10) zwangsweise beendet wird, wenn eine vorgegebene Grenzmenge Vorfüllflüssigkeit durch den Dialysator (28) hindurch ultrafiltriert wurde.

4. Hämodialysegerät (10) nach einem der vorangegangenen Ansprüche, wobei während der Vorfüllflüssigkeits- Entfernung die Blutpumpe (40) durch das Hämodialysegerät (10) mit einer Pumprate betrieben wird, die eine ungefähr gleichzeitige Blutankunft aus den beiden blutseitigen Leitungen (42,44) an der Dialysator- Membran (31) gewährleistet.

5. Hämodialysegerät (10) nach einem der vorangegangenen Ansprüche, wobei die Vorfüllflüssigkeits- Quelle (16) eine Dialysatquelle (16) ist und beim Vorfüllen die Vorfüllflüssigkeit von der Dialysatpumpe (22) durch die Dialysator- Membran (31) in die arterielle und die venöse Leitung (42,44) gepumpt wird.

6. Hämodialysegerät (10) nach einem der vorangegangenen Ansprüche, wobei die Dialysatpumpe (22) zwangsweise mit einer Abfallpumpe (24) zu einer Bilanzierpumpe (20) gekoppelt ist, und als Saugpumpe (34) eine separate Ultrafiltrationspumpe (34) fluidisch parallel zu der Abfallpumpe (24) vorgesehen ist, wobei die Ultrafiltrationspumpe (34) beim Vorfüllen im Gegenstrom und bei der Ultrafiltration im Parallelstrom als Saugpumpe (34) arbeitet.

7. Hämodialysegerät (10) nach einem der vorangegangenen Ansprüche, wobei im Verlauf der venösen Leitung (44) eine Luftfalle (56) angeordnet ist, wobei während der Vorfüllflüssigkeits- Entfernung durch das Hämodialysegerät (10) der Flüssigkeitspegel in der Luftfalle (56) oberhalb einer Einlassöffnung (59) einer Einlaufleitung (55) gehalten wird.

## Claims

1. Hemodialysis apparatus (10) comprising:
a pre-filling liquid source (16) providing a pre-filling liquid,
a dialysate side (12) comprising a dialysate chamber (29) of a dialyzer (28) which has a membrane (31), a dialysate pump (22) which pumps dialysate to the dialysate chamber (29), a suction pump (34) which pumps the dialysate or the pre-filling liquid away from the dialysate chamber (29), and a dialysate pressure sensor (32) between the dialysate chamber (29) and a waste pump (24), and
a blood side (14) comprising an arterial line (42), a blood pump (40), a blood chamber (30) of the dialyzer (28) and a venous line (44),
wherein the hemodialysis apparatus (10)
with the arterial and venous lines (42, 44) being pre-filled with pre-filling liquid from the pre-filling liquid source (16),
with the arterial and venous lines (42, 44) each being applied to a patient (50),
and after manual triggering of the removal of the pre-filling liquid,
performs the following method steps for starting a hemodialysis:
pre-filling liquid removal by ultrafiltration of the pre-filling liquid from the arterial and venous lines (42, 44) via the dialyzer membrane (31) into the dialysate chamber (29) of the dialyzer (28) by the suction pump (34), and during the pre-filling liquid removal: continuous determination of the fluid pressure by the dialysate pressure sensor (32), wherein the pre-filling liquid removal is stopped automatically, if the fluid pressure measured by the dialysate pressure sensor (32) falls below a specified blood arrival limit pressure.

2. Hemodialysis apparatus (10) of claim 1, comprising the method step, after stopping the pre-filling liquid removal: starting the dialysis mode by the hemodialysis apparatus (10) at a penetration rate different from the penetration rate of the pre-filling liquid removal.

3. Hemodialysis apparatus (10) of one of the preceding claims, wherein the pre-filling liquid removal is forcibly stopped by the hemodialysis apparatus (10) when a predetermined limit quantity of pre-filling liquid has been ultrafiltrated through the dialyzer (28).

4. Hemodialysis apparatus (10) of one of the preceding claims, wherein during the pre-filling liquid removal, the blood pump (40) is operated by the hemodialysis apparatus (10) at a pumping rate which guarantees an approximately simultaneous blood arrival from the two blood-side lines (42, 44) to the dialyzer membrane (31).

5. Hemodialysis apparatus (10) of one of the preceding claims, wherein the pre-filling liquid source (16) is a dialysate source (16) and, during pre-filling, the pre-filling liquid is pumped by the dialysate pump (22) through the dialyzer membrane (31) into the arterial and venous lines (42, 44).

6. Hemodialysis apparatus (10) of one of the preceding claims, wherein the dialysate pump (22) is forcibly coupled to the waste pump (24) to form a balancing pump (20), and a separate ultrafiltration pump (34) is provided as a suction pump (34) fluidically parallel to the waste pump (24), wherein the ultrafiltration pump (34) operates as a suction pump (34) in counterflow during pre-filling and in parallel flow during ultrafiltration.

7. Hemodialysis apparatus (10) of one of the preceding claims, wherein an air trap (56) is arranged along the venous line (44), wherein the hemodialysis apparatus (10) maintains the pre-filling liquid level in the air trap (56) above an inlet opening (59) of an inlet line (55) during pre-filling liquid removal.

## Revendications

1. Dispositif d'hémodialyse (10) ayant:
une source de liquide de pré-remplissage (16) fournissant un liquide de pré-remplissage,
un côté dialysat (12) comprenant une chambre de dialysat (29) d'un dialyseur (28) qui comprend une membrane (31), une pompe de dialysat (22) qui pompe le dialysat vers la chambre de dialysat (29), une pompe d'aspiration (34) qui pompe le dialysat ou le liquide de pré-remplissage den de la chambre de dialysat (29), et un capteur de pression de dialysat (32) entre la chambre de dialysat (29) et une pompe de déchets (24), et
un côté sang (14) comprenant une ligne artérielle (42), une pompe à sang (40), une chambre à sang (30) du dialyseur (28) et une ligne veineuse (44),
le dispositif d'hémodialyse (10),
avec les lignes artérielle et veineuse (42, 44) pré-remplies avec du liquide de pré-remplissage venant de la source de liquide de pré-remplissage (16),
avec chacune des lignes artérielle et veineuse (42, 44) appliquées à un patient (50),
et après l'initialisation manuel de l'enlèvement du liquide de pré-remplissage,
met à exécution les étapes de procédé suivantes pour démarrer une hémodialyse:
enlèvement du liquide de pré-remplissage par ultrafiltration du liquide de pré-remplissage des lignes artérielle et veineuse (42, 44) à travers la membrane du dialyseur (31) dans la chambre de dialysat (29) du dialyseur (28) par la pompe d'aspiration (34), et
pendant l'enlèvement du liquide de pré-remplissage: détermination continue de la pression de fluide par le capteur de pression de dialysat (32), l'enlèvement du liquide de pré-remplissage étant arrêtée automatiquement lorsque la pression de fluide mesurée par le capteur de pression de dialysat (32) n'atteint pas une pression limite d'arrivée de sang spécifiée.

2. Dispositif d'hémodialyse (10) selon la revendication 1, comprenant, après l'arrêt de l'enlèvement du liquide de pré-remplissage, l'étape suivante: démarrer le mode de dialyse par le dispositif d'hémodialyse (10) à une vitesse de pénétration différente de la vitesse de pénétration d'enlèvement de liquide de pré-remplissage.

3. Dispositif d'hémodialyse (10) selon l'une quelconque des revendications précédentes, dans lequel l'enlèvement du liquide de pré-remplissage est arrêté forcément par le dispositif d'hémodialyse (10) lorsqu'une quantité limite prédéterminée de liquide de pré-remplissage a été ultrafiltrée à travers le dialyseur (28).

4. Dispositif d'hémodialyse (10) selon l'une quelconque des revendications précédentes, dans lequel, pendant l'enlèvement de liquide de pré-remplissage, la pompe de sang (40) est opérée par le dispositif d'hémodialyse (10) à une vitesse de pompage garantissant une arrivée de sang approximativement simultanée des deux lignes côté sang (42, 44) vers la membrane du dialyseur (31).

5. Dispositif d'hémodialyse (10) selon l'une quelconque des revendications précédentes, dans lequel la source de liquide de pré-remplissage (16) est une source de dialysat (16) et le liquide de pré-remplissage est pompé par la pompe de dialysat (22) à travers la membrane de dialysat (31) dans les lignes artérielle et veineuse (42, 44) pendant le pré-remplissage.

6. Dispositif d'hémodialyse (10) selon l'une quelconque des revendications précédentes, dans lequel la pompe de dialysat (22) est couplée forcément à la pompe à déchets (24) pour former une pompe d'équilibrage (20), et en tant de pompe d'aspiration (34), une pompe d'ultrafiltration (34) séparée est prévue fluidiquement parallèle à la pompe à déchets (24), la pompe d'ultrafiltration (34) fonctionnant comme une pompe d'aspiration (34) à contre-courant pendant pré-remplissage et à courant parallèle pendant ultrafiltration.

7. Dispositif d'hémodialyse (10) selon l'une quelconque des revendications précédentes, dans lequel un piège à air (56) est agencé au cours de la ligne veineuse (44), le dispositif d'hémodialyse (10) maintenant le niveau de liquide dans le piège à air (56) au-dessus d'une ouverture d'entrée (59) d'une ligne d'entrée pendant l'enlèvement de liquide de pré-remplissage (55).
